# EUROPEAN PATENT SPECIFICATION

(11) **EP 2 749 237 B1**
(45) Date of publication and mention of the grant of the patent: **08.03.2017**
(21) Application number: 13199143.2
(22) Date of filing: 20.12.2013
(51) Int. Cl.: A61B 17/72, A61B 17/86

(54) **Hammertoe compression device having two threaded ends**
Hammerzehkompressionsvorrichtung mit zwei Gewindeenden
Dispositif de compression pour orteil en marteau à deux extrémités filetées

(30) Priority: 27.12.2012 US 201261746320 P; 09.12.2013 US 201314100829
(43) Date of publication of application: 02.07.2014
(73) Proprietor: Wright Medical Technology, Inc., Arlington, Tennessee 38002 (US)
(72) Inventor: Cummings, Shannon, Hernando, Mississippi 38632 (US)
(74) Representative: Gerbino, Angelo

(56) References cited:
- EP-A1- 2 564 799
- EP-A2- 1 870 050
- WO-A1-2013/096746
- US-A- 4 723 541
- US-A- 5 019 079
- US-A1- 2011 082 508
- US-A1- 2012 271 362
- US-A1- 2013 030 475

## Description

### FIELD OF DISCLOSURE

The disclosed device and method generally relate to hammertoe correction implants and devices. More specifically, the present invention relates to a bone implant having the features disclosed in the preamble of claim 1 which follows. A bone implant of this kind is disclosed in the prior art document US 4,723,541 A .

### BACKGROUND

A hammertoe or contracted toe is a deformity of the proximal inter-phalangeal joint of the second, third, or fourth toe causing it to be permanently bent and giving it a semblance of a hammer. Initially, hammertoes are flexible and may be corrected with simple measures but, if left untreated, hammertoes may require surgical intervention for correction. Persons with hammertoe may also have corns or calluses on the top of the middle joint of the toe or on the tip of the toe and may feel pain in their toes or feet while having difficulty finding comfortable shoes.

Various treatment strategies are available for correcting hammertoes. Conventionally, the first line of treatment for hammertoes includes employing new shoes having soft and spacious toe boxes. Additionally, toe exercises may be prescribed to stretch and strengthen respective muscles, e.g., gently stretching one's toes manually, using the toes to pick up things off the floor, etc. Another line of treatment may include employing straps, cushions or non-medicated corn pads to relieve symptoms. An addition method of treatment may include correction by surgery if other non-invasive treatment options fail. Conventional surgery usually involves inserting screws, wires or other similar implants in toes to straighten them. Traditional surgical methods generally include the use of Kirschner wires (K-wires). Due to various disadvantages of using K-wires, however, compression screws are being employed as a better implant alternative as K-wires require pings protruding
through the end of respective toes due to their temporary nature. As a result, K-wires often lead to pin tract infections, loss of fixation, and other conditions. Additional disadvantages of K-wires include migration and breakage of the K-wires thus resulting in multiple surgeries.

Screw implants, however, may provide a more permanent solution as such implants do not need removal and thus have no protruding ends. Further, with the use of screw implants, a patient may wear normal footwear shortly after the respective surgery. Conventional screw implants possess a completely threaded body and do not provide a flexibility to the respective toe in its movement, i.e., conventional implants provide a pistoning effect. Furthermore, conventional screw implants are made for strong bones and are unsuitable for treatment of patients having weak bones which is a predominant reason why K-wire surgical implants are still employed despite their several disadvantages. Accordingly, there remains a need for developing hammertoe implants and devices including compression screw which are not only stable but provide adequate compression across a joint.

### BRIEF DESCRIPTION OF THE DRAWINGS

Other objects, features, and advantages of the present invention will be apparent from the following description when read with reference to the accompanying drawings. In the drawings, wherein like reference numerals denote corresponding parts throughout the several views.
Figure 1 illustrates a front plan view of an exemplary implant according to some embodiments of the present subject matter.
Figure 2 is an illustration of an exemplary driver according to some embodiments of the present subject matter.
Figure 3 is an illustration of the drilling of the middle and proximal phalanxes of the foot.
Figure 4 is an illustration of the installation of an exemplary implant into a proximal phalanx of the foot.
Figure 5 is an illustration of the passing of the driver through the middle and distal phalanxes of the foot.
Figure 6 is an illustration of alignment of the middle, proximal and distal phalanxes of the foot with a re-engagement of the driver to an installed implant.

### DETAILED DESCRIPTION

With reference to the figures, where like elements have been given like numerical designations to facilitate an understanding of the present subject matter, the various embodiments of a double thread hammertoe compression device are described.

It should be noted that the figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures. The terms "implant" and "device" are used interchangeably in this disclosure.

Embodiments of the present subject matter provide a surgeon stability and compression across proximal or distal interphalangeal joints while maintaining simplicity of a hammertoe fusion. Exemplary embodiments feature a double-ended threaded device, each end having a pitch (disparate or otherwise) that, when implanted, provides compression across a targeted joint.

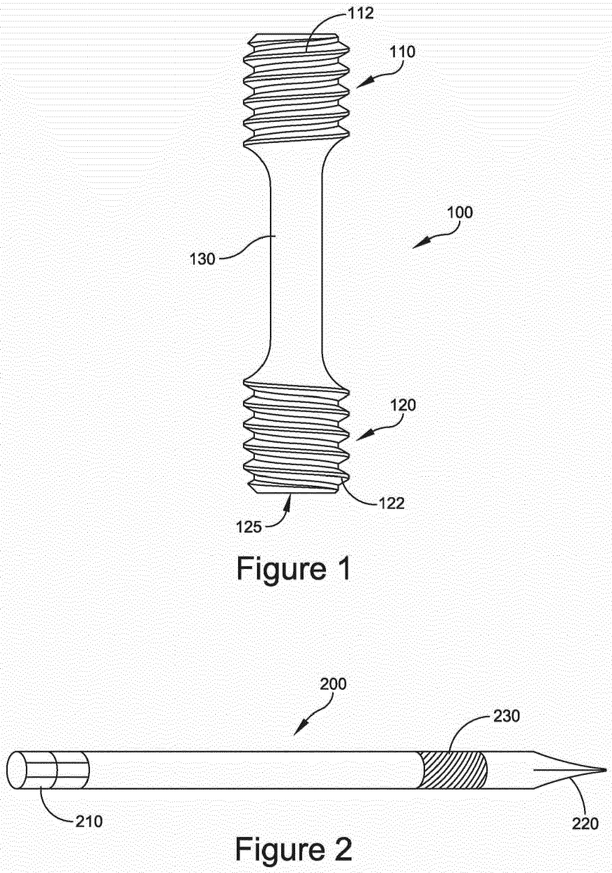

Figure 1 illustrates a front plan view of an exemplary implant according to some embodiments of the present subject matter. With reference to Figure 1, an implant 100 for correcting hammertoes may comprise a proximal end 110 and a distal end 120 connected by a solid core 130 or rod. The proximal end 110 includes threads 112 on an external surface thereof having a first pitch, and the distal end 120 includes threads 122 on an external surface thereof having a second pitch. The threads 112 on the proximal end 110 have a pitch of .039, and the threads 122 on the distal end 120 have a pitch of .049. Further, the thread pitches are threaded in opposing directions. The implant 100 may be constructed of any suitable material such as stainless steel, titanium, or other metals or rigid polymers. In one embodiment, the distal end 120 may include a female depression 125 adaptable to mate with a driver (not shown) having a male extension. Of course, the distal end 120 may have any suitable type of interfacing mechanism to accept conventional implant drivers such as a screw head or the like. For example, the distal end 120 may have a portion in the shape of a hex whereby a suitable driver has a corresponding hex adapter appropriate to drive the implant 100 into a respective bone.

Exemplary implants 100 may be implanted into targeted bones by conventional methods. For example, an exemplary implant 100 may be implanted or installed via a retrograde approach between, for example, proximate and middle phalanxes in a foot. One skilled in the art will understand that the method described herein may be applied to the middle and distal phalanxes as well or other adjacent bones. Figure 2 is an illustration of an exemplary driver according to some embodiments of the present subject matter. With reference to Figure 2, an exemplary driver 200 may be an elongated instrument and include one end having an adaptable portion 210 suitable for mating with an implant 100 described above. In the illustrated example, the adaptable portion 210 comprises a male hexagonal head adaptable to mate to a corresponding female depression in an implant. In one embodiment of the present subject matter, the male hexagonal head is a 2.0 mm hexagonal head. Of course, other geometries and interfacing mechanisms are envisioned and the male hexagonal head of the driver 200 and its noted dimensions should not limit the scope of the claims appended herewith. On an opposing end of the driver 200 may be a driving pin 220 or trocar and may include a flat modular section 230 adaptable to accept a handle or other suitable mechanism to assist a surgeon during installation of an exemplary implant 100.

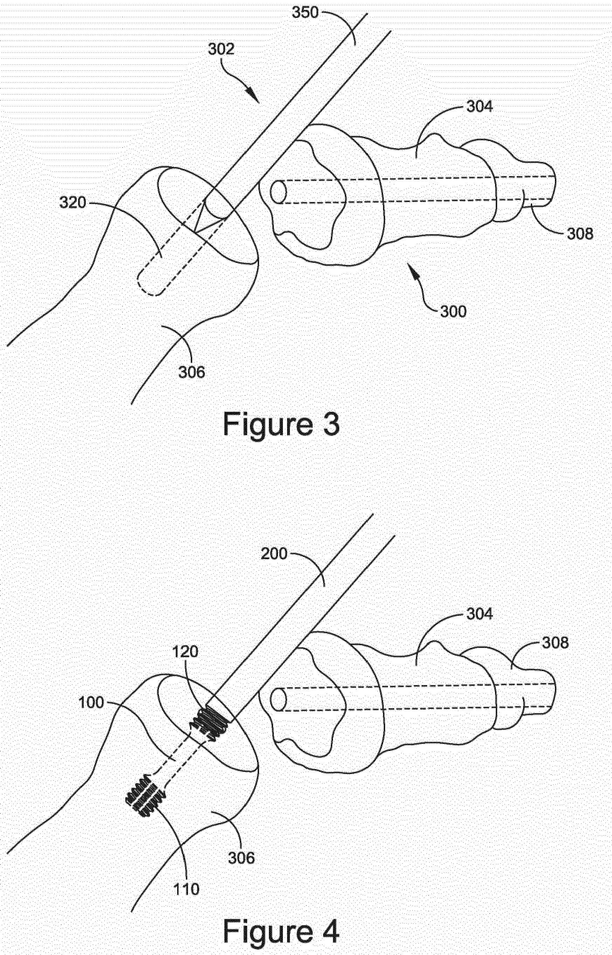

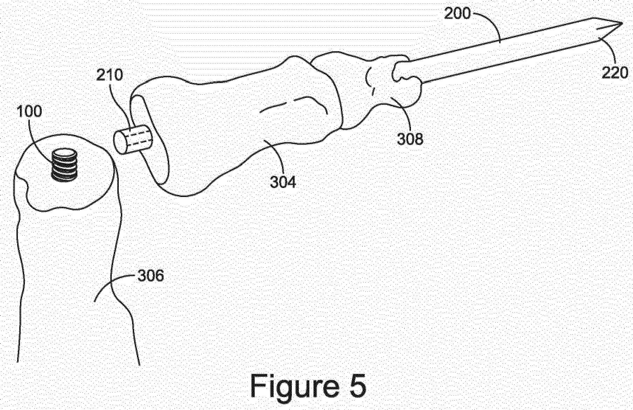

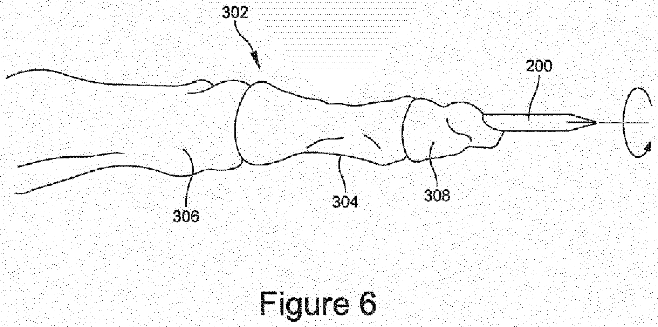

Figures 3-6 illustrate an exemplary method of installation or implantation of an implant according to embodiments of the present subject matter. With reference to Figures 3-6, in one embodiment to install an implant a toe 300 may be opened to provide access to a joint 302 between a middle phalanx 304 and a proximal phalanx 306. The middle and proximal phalanxes 304, 306, respectively, may be resected using a bone saw or other tool 350, if necessary. An intermedullary canal 320 may be drilled into both the middle and proximal phalanxes 304, 306 using a drill 350 or other mechanism to an appropriate depth. A driver 200 may be engaged with the distal end 120 of an exemplary implant 100 as described above, and the proximal end 110 of the implant 100 may be threaded into the proximal phalanx 306 until the threads of the distal end 120 of the implant sits flush to the surface of the proximal phalanx 306. The driver 200 may then be loosened and removed from the implant 100 and the driver retrograded distally until the pin or trocar end of the driver 200 passes through the middle phalanx 304 and out of the distal tip of the distal phalanx 308 as illustrated in Figure 5. The retrograde of the driver 200 may terminate when the adaptable portion of the driver 200 reaches the surface of the middle phalanx 304. The joint 302 may then be closed and the driver 200 re-engaged with the distal end 120 of the implant. Upon engagement of the driver 200 with the implant 100 the implant may be driven distally into the middle phalanx 304. As the distal end 120 of the implant 100 is driven, i.e., rotated about its respective longitudinal axis, into the middle phalanx 304, compression of the respective joint 302 is created by the distal threads of the implant travelling faster than the proximal threads of the implant. Compression of the joint may also be effected as a function of the disparate thread pitches of the implant and/or the directions of the thread pitches of the implant.

Although reference has been made to a patient's proximal and distal interphalangeal joints metatarsal phalangeal joint, one skilled in the art will understand that embodiments of the present subject matter may be implemented for other respective bones including, but not limited to other phalanges/digits and phalangeal/digital joints.

It may be emphasized that the above-described embodiments, particularly any "preferred" embodiments, are merely possible examples of implementations and merely set forth for a clear understanding of the principles of the disclosure. Many variations and modifications may be made to the above-described embodiments of the disclosure without departing substantially from the spirit and principles of the disclosure. All such modifications and variations are intended to be included herein within the scope of this disclosure.

While this specification contains many specifics, these should not be construed as limitations on the scope of the claimed subject matter, but rather as descriptions of features that may be specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable sub-combination.

As shown by the various configurations and embodiments illustrated in Figures 1-6, a double thread hammertoe compression device has been described.

While preferred embodiments of the present subject matter have been described, it is to be understood that the embodiments described are illustrative only and that the scope of the invention is to be defined solely by the appended claims many variations and modifications naturally occurring to those of skill in the art from a perusal hereof.

## Claims

1. A bone implant (100) comprising:
an elongated device having a first threaded end (110) with a first thread pitch and a second threaded end (120) with a second thread pitch; and
a core (130) connecting the first and second threaded ends (110, 120),
wherein the implant (100) is adapted to be implanted into a joint (302), and
wherein when the implant (100) is rotated about its longitudinal axis by a driver (200) the joint (302) is compressed using the first thread pitch on a distal bone in the joint (302) and the second thread pitch on a proximate bone in the joint (302),
wherein said bone implant (100) is unitary, wherein the first and second thread pitches are in opposing directions,
**characterised in that** the second threads of the implant (100) travel faster than the first threads of the implant (100), wherein the first thread pitch is of 0.039 and the second thread pitch is of 0.049.

2. The bone implant (100) of Claim 1 wherein the distal bone is a phalange (304) selected from the group consisting of proximal phalange, intermediate phalange, distal phalange, and combinations thereof.

3. The bone implant (100) of Claim 1 wherein the proximate bone is a phalange (306) selected from the group consisting of proximal phalange, intermediate phalange, distal phalange, and combinations thereof.

## Patentansprüche

1. Knochenimplantat (100), umfassend:
eine längliche Vorrichtung, welche ein erstes Gewindeende (110) mit einer ersten Gewindesteigung und ein zweites Gewindeende (120) mit einer zweiten Gewindesteigung hat; und
einen Kern (130), welcher das erste und das zweite Gewindeende (110, 120) verbindet,
wobei das Implantat (100) dafür ausgebildet ist, in ein Gelenk (302) implantiert zu werden, und
wobei, wenn das Implantat (100) durch einen Schraubendreher (200) um seine Längsachse gedreht wird, das Gelenk (302) unter Anwendung der ersten Gewindesteigung auf einen distalen Knochen in dem Gelenk (302) und der zweiten Gewindesteigung auf einen proximalen Knochen in dem Gelenk (302) gedrückt wird,
wobei das Knochenimplantat (100) einstückig ist,
wobei die erste und die zweite Steigung in einander abgewandte Richtungen verlaufen,
**dadurch gekennzeichnet, dass** die zweiten Gewindegänge des Implantats (100) schneller als die ersten Gewindegänge des Implantats (100) laufen, wobei die erste Gewindesteigung 0,039 beträgt und die zweite Gewindesteigung 0,049 beträgt.

2. Knochenimplantat (100) nach Anspruch 1, wobei der distale Knochen ein Zehenglied (304) ist, welches aus der Gruppe bestehend aus proximalem Zehenglied, mittlerem Zehenglied, distalem Zehenglied und Kombinationen daraus gewählt ist.

3. Knochenimplantat (100) nach Anspruch 1, wobei der proximale Knochen ein Zehenglied (306) ist, welches aus der Gruppe bestehend aus proximalem Zehenglied, mittlerem Zehenglied, distalem Zehenglied und Kombinationen daraus gewählt ist.

## Revendications

1. Implant osseux (100) comprenant :
un dispositif allongé comprenant une première extrémité filetée (110) présentant un premier pas de filetage et une seconde extrémité filetée (120) présentant un second pas de filetage ; et
une partie centrale (130) raccordant les première et seconde extrémités filetées (110, 120),
dans lequel l'implant (100) est adapté à être implanté dans une articulation (302), et
dans lequel, lorsque l'implant (100) est entraîné en rotation autour de son axe longitudinal par un élément d'entraînement (200), l'articulation (302) est comprimée au moyen du premier pas de filetage sur un os distal dans l'articulation (302) et du second pas de filetage sur un os proximal dans l'articulation (302),
dans lequel ledit implant osseux (100) est unitaire, dans lequel les premier et second pas de filetage sont dans des directions opposées,
**caractérisé en ce que** les seconds filetages de l'implant (100) se déplacent plus vite que les premiers filetages de l'implant (100), dans lequel le premier pas de filetage est de 0,039 et le second pas de filetage est de 0,049.

2. Implant osseux (100) selon la revendication 1 dans lequel l'os distal est une phalange (304) sélectionnée parmi le groupe constitué d'une phalange proximale, d'une phalange intermédiaire, d'une phalange distale, et de combinaisons de celles-ci.

3. Implant osseux (100) selon la revendication 1 dans lequel l'os proximal est une phalange (306) sélectionnée parmi le groupe constitué d'une phalange proximale, d'une phalange intermédiaire, d'une phalange distale, et de combinaisons de celles-ci.
